# EUROPÄISCHE PATENTSCHRIFT

(11) **EP 3 856 276 B1**
(45) Veröffentlichungstag und Bekanntmachung des Hinweises auf die Patenterteilung: **21.02.2024**
(21) Anmeldenummer: 19783453.4
(22) Anmeldetag: 26.09.2019
(51) Int. Cl.: A61M 60/126, A61M 60/216, A61M 60/824, F04D 29/047, F04D 29/02

(54) **GEKAPSELTE MIKROPUMPE**
SEALED MICROPUMP
MICROPOMPE ENCAPSULÉE

(30) Priorität: 28.09.2018 DE 102018216695
(43) Veröffentlichungstag der Anmeldung: 04.08.2021
(73) Patentinhaber: Kardion GmbH, 70376 Stuttgart (DE)
(72) Erfinder: STOTZ, Ingo, 71254 Ditzingen (DE); BETTE, Johannes, 71229 Leonberg (DE); MINZENMAY, David, 70569 Stuttgart (DE); EIBERGER, Fabian, 70839 Gerlingen (DE)
(74) Vertreter: Pfiz, Thomas
(86) Internationale Anmeldenummer: PCT/EP2019/076002
(87) Internationale Veröffentlichungsnummer: WO 2020/064911

(56) Entgegenhaltungen:
- WO-A1-2010/119267
- WO-A1-2013/173239
- US-A- 4 846 152
- US-A1- 2001 041 934

## Beschreibung

Die vorliegende Erfindung betrifft eine gekapselte Mikropumpe mit integriertem Motor und wenigstens einem Laufrad zur Erzeugung eines Fluidflusses innerhalb eines Gehäuses der Mikropumpe.

Zur Herz-Kreislauf-Unterstützung bei herzinsuffizienten Patienten werden Systeme eingesetzt, die einen Teil der Pumpfunktion oder sogar die komplette Pumpfunktion des Herzens übernehmen. Neben außen am Herz aufsitzenden Systemen, die invasiv implantiert werden, sind auch Systeme bekannt, die als mechanische Pumpen minimalinvasiv insbesondere in den linken Ventrikel des Herzens und die sich daran anschließende Aorta eingesetzt werden. Durch kontinuierliches Pumpen wird Blut aus der linken Herzkammer in die Aorta gepumpt, so dass bei einem herzinsuffizienten Patienten ausreichend sauerstoffreiches Blut in den Körper gelangt. Beispielsweise beschreibt die US-Patentanmeldungsschrift US 2013/0303833 A1 eine implantierbare Blutpumpe, bei der durch einen im Inneren angeordneten Rotor ein Blutfluss innerhalb des Gehäuses erzeugt wird.

US 2001/0041934 A1 offenbart ein künstliches Herz mit einer Axialstrompumpe zum Fördern eines Blutstroms. Die Axialstrompumpe hat eine Antriebswelle, die mit einem keramischen Lager und mit einem keramischen Staudrucklager gelagert ist.

WO 2010/119267 A1 zeigt eine Rotationspumpe zum Implantieren in das menschliche Herz oder Gefäßsystem.

WO 2013/173239 A1 beschreibt eine Katheterpumpe mit einer expandierbaren Kanüle, wobei innerhalb der Kanüle ein Laufradanordnung zum Ansaugen von Blut angeordnet ist.

US 4,846,152 A offenbart eine intravaskuläre Hochgeschwindigkeits-Blutpumpe mit einem hydrostatischen Lager.

Durch die minimalinvasive Implantation müssen diese Systeme insbesondere im Hinblick auf deren radialen Umfang sehr klein ausgestaltet werden, beispielsweise mit einem Außendurchmesser von maximal 10 mm. Diese Anforderungen an eine Miniaturisierung der Komponenten stellen eine große Herausforderung dar.

Ausgehend hiervon liegt der Erfindung die Aufgabe zugrunde, die im Stand der Technik bekannten Mikropumpen und Systeme weiter zu verbessern und möglichst hohe Wirkungsgrade in der Fluid- bzw. Blutförderung bei hoher Zuverlässigkeit und Lebensdauer zu erreichen.

Die Erfindung , die sich auf die beigefügten Ansprüche beschränkt, geht von Überlegungen aus, für minimalinvasive Implantationsanwendung Blutpumpen einzusetzen, die auf dem Prinzip einer Kreiselpumpe mit einem integrierten Elektromotor für den Antrieb basieren. Hierbei wird mittels eines Laufrades der geforderte Blutfluss erzeugt. Bei solchen Systemen sollte der Motor vollständig gekapselt ausgeführt sein. Das Drehmoment kann dabei über eine permanentmagnetische Kupplung (Radialdrehkupplung) berührungslos übertragen werden. Dabei ist es in der Regel erforderlich, das Laufrad radial und axial zu lagern. Für die radiale Lagerung kann gehäuseseitig ein sogenannter Lagerstern vorgesehen sein, der Aussparungen beziehungsweise Löcher für den Fluiddurchfluss aufweist.

Die Erfindung stellt eine gekapselte Mikropumpe mit integriertem Motor und mit wenigstens einem Laufrad zur Erzeugung eines Fluidflusses innerhalb eines Gehäuses der Mikropumpe bereit, wobei die Pumpe ein radiales Gleitlager mit einem Lagerstern zur Lagerung eines Laufradzapfens des Laufrades der Pumpe innerhalb des Gehäuses aufweist. Kernpunkt der Erfindung ist, dass der Laufradzapfen eine Ummantelung aus einem anderen Material als der Lagerstern aufweist. Die Ummantelung ist fest mit dem Laufradzapfen verbunden. Durch diese Ausgestaltung können auf der einen Seite tribologisch vorteilhafte Materialpaarungen für dieses Gleitlager realisiert werden und auf der anderen Seite wird eine Raumeinsparung für die Lagerung des Laufradzapfens ermöglicht. Diese Raumeinsparung kann im Vergleich mit bereits bekannten Mikropumpen zugunsten einer Vergrößerung der Aussparungen im den den Laufradzapfen umgebenden Lagerstern genutzt werden. Dies hat den erheblichen Vorteil, dass hierdurch Druckverluste in diesem Bereich verringert werden.

Der für das radiale Gleitlager erforderliche Raumbedarf stellt zwingenderma-βen einen Strömungswiderstand dar. Die erfindungsgemäße Lösung ermöglicht es, die Öffnungen für den Fluiddurchfluss in dem Lagerstern zu vergrö-βern, da insbesondere auf eine Lagerbuchse innerhalb der zentralen Öffnung des Lagersterns verzichtet werden kann. Die reibungstechnisch vorteilhafte Vermeidung einer direkten Materialpaarung zwischen dem Laufradzapfen und dem Lagerstern, die üblicherweise beide aus metallischen Werkstoffen, beispielsweise aus Titan, gefertigt sind, durch ein dazwischenliegendes Bauteil aus einem anderen Werkstoff, insbesondere aus Kunststoff, wird durch die erfindungsgemäß vorgesehene Ummantelung des Laufradzapfens mit einem solchen anderen Material realisiert. Insgesamt können dadurch Druckverluste am radialen Gleitlager minimiert werden, wodurch der Wirkungsgrad der Pumpe gesteigert wird. Weiterhin sind kleinere Drehzahlen beim Betrieb der Mikropumpe möglich, wodurch insgesamt weniger Lagerverschleiß stattfindet und auch eine schonendere Förderung für das zu fördernde Fluid möglich ist, so dass beispielsweise im Fall einer intravasalen Blutpumpe weniger Schädigung der empfindlichen Blutkomponenten auftritt. Insgesamt ermöglicht die Ausgestaltung der gekapselten Mikropumpe und insbesondere des hierin vorgesehenen radialen Gleitlagers für das Laufrad einen deutlich verringerten Strömungswiderstand für das Fluid, ohne die Zuverlässigkeit und Lebensdauer der Mikropumpe zu beeinträchtigen.

Für die Herstellung der beschriebenen gekapselten Mikropumpe wird als Material für den Laufradzapfen und für den Lagerstern vorteilhafterweise ein metallischer Werkstoff gewählt und die Ummantelung des Laufradzapfens ist aus einem Kunststoff gefertigt. Insbesondere können der Laufradzapfen und der Lagerstern aus Titan gefertigt sein. Die Ummantelung des Laufradzapfens ist bevorzugterweise aus Polyetheretherketon (PEEK).

In einer besonders bevorzugten Ausgestaltung der gekapselten Mikropumpe ist der Laufradzapfen im Bereich der Ummantelung im Querschnitt verjüngt. Hierdurch kann in besonders vorteilhafter Weise eine Vergrößerung des zur Verfügung stehenden Bauraumes für den Lagerstern realisiert werden. Durch die Verjüngung des Laufradzapfens kann die Ummantelung des Laufradzapfens im Lagerbereich so gestaltet werden, dass gegenüber dem Bereich des Laufradzapfens außerhalb des Lagers keine Vergrößerung des radialen Querschnitts auftritt. Dies wirkt sich auf den Lagerstern derart aus, dass der Platzbedarf für die bei herkömmlichen Lösungen vorgesehene Lagerbuchse im Lagerstern vollständig zur Verfügung steht, so dass der Lagerstern und damit dessen Durchtrittsöffnungen für das Fluid entsprechend größer ausgelegt werden können.

Die Ummantelung des Laufradzapfens bildet eine Kappe, die über den Lagerstern hinaus eine Verlängerung aufweist. Vorzugsweise verjüngt sich diese Verlängerung in stromaufwärtiger Richtung, wodurch eine strömungstechnisch sehr vorteilhafte Form realisiert wird, die durch eine Reduktion von Ablösegebieten und eine Verringerung des Staubereichs eine bessere Strömungsführung um das Lager herum ermöglicht und dadurch zur Reduzierung von Druckverlusten und zur Steigerung der Effizienz der Mikropumpe beiträgt. Für die Verlängerung der Kappe können dabei insbesondere konische oder halbellipsoide Formen bevorzugt sein.

Bei der gekapselten Mikropumpe kann es sich mit besonderem Vorteil um eine Blutpumpe, insbesondere um eine intravasale Blutpumpe für mikroinvasive Anwendungen handeln. Beispielsweise kann es sich bei der Mikropumpe um den Bestandteil eines ventrikulären Herzunterstützungssystems handeln. Die hier vorgeschlagenen Verbesserungen der Mikropumpe kommen insbesondere bei Systemen mit sehr kleinem Bauraum zum Tragen, so dass die Vorteile der hier beschriebenen Mikropumpe für intravasale Blutpumpen eine besondere Rolle spielen. Vorzugsweise beträgt der Außendurchmesser der hier beschriebenen gekapselten Mikropumpe 10 mm oder weniger, so dass damit den Anforderungen an minimalinvasiv zu implantierende Blutpumpen in vollem Umfang Rechnung getragen wird.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

In den Zeichnungen zeigen:
- Fig. 1: eine aktuelle Entwicklung einer gekapselten Mikropumpe mit integriertem Motor (Teilausschnitt in Längsschnitt) als Ausgangspunkt der Erfindung;
- Fig. 2: einen Längsschnitt durch ein radiales Gleitlager eines Laufradzapfens der gekapselten Mikropumpe aus Fig. 1;
- Fig. 3: einen Längsschnitt durch ein radiales Gleitlager eines Laufradzapfens einer gekapselten Mikropumpe in einer bevorzugten Ausführungsform der Erfindung und
- Fig. 4: vergleichende Querschnitte durch die radialen Gleitlager der Fig. 2 (4A) und der erfindungsgemäßen Ausführungsform eines Gleitlagers aus Fig. 3 (4B).

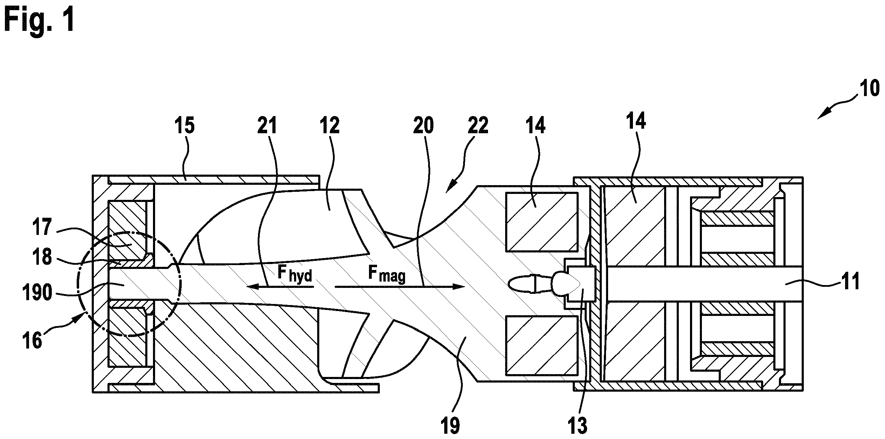

Fig. 1 zeigt im Querschnitt den hydraulisch aktiven Teil einer vollständig gekapselten Mikropumpe 10 gemäß einer aktuellen Entwicklung solcher Pumpen. Diese Mikropumpe 10 ist insbesondere als Blutpumpe für minimalinvasive Implantationen (intravasale Blutpumpe) vorgesehen. Die Mikropumpe 10 wird von einem integrierten Elektromotor angetrieben, von dem hier die Motorwelle 11 gezeigt ist. Der Rotor bzw. das Laufrad 19 mit den Laufradblättern (Schaufeln) 12 ist über ein Spitzenlager 13 radial und axial gelagert, wobei das Drehmoment über eine permanentmagnetische Kupplung 14 übertragen wird. Mittels des Laufrades 19 wird der geforderte Blutfluss innerhalb des Gehäuses 15 der gekapselten Blutpumpe 10 erzeugt. Das Laufrad 19 bildet dabei gewissermaßen einen von einem Gehäuse umschlossenen Propeller (Impeller). Der Pfeil 20 deutet die magnetisch wirkenden Kräfte an. Der Pfeil 21 deutet die hydraulisch wirksamen Kräfte an. Der Laufradzapfen 190 (Lagerzapfen) des Laufrades 19 ist zusätzlich über ein radiales Gleitlager 16, welches stromaufwärts liegt, gelagert. Das radiale Gleitlager 16 umfasst einen Lagerstern 17 mit einer darin eingebrachten Lagerbuchse 18 und dem innerhalb der Lagerbuchse 18 rotierenden Laufradzapfen 190. Die Lagerbuchse 18 ist vorgesehen, um eine reibungstechnisch ungünstige Materialpaarung zwischen dem Laufradzapfen 190 und dem Lagerstern 17 zu vermeiden, beispielsweise die Materialpaarung Titan-Titan, die mit einem hohen Verschleiß verbunden ist. So kann die Lagerbuchse 18 aus Polyetheretherketon (PEEK) gefertigt sein, so dass zwischen der Lagerbuchse 18 und dem Laufradzapfen 190 die tribologisch vorteilhafte Materialpaarung PEEK-Titan vorliegt, die sehr reibungs- und verschleißarm ist.

Durch die Rotation des Laufrades 19 wird der Blutfluss innerhalb des Gehäuses 15 erzeugt. Der Lagerstern 17 weist mehrere Eintrittsöffnungen für das Blut auf. Dennoch sind im Bereich des Lagersterns Druckverluste vorhanden, da durch den Lagerstern 17 der Querschnitt eingeengt wird und eine Staustelle gebildet wird. Im Bereich der Basis des Laufrades 19 befinden sich Öffnungen 22 im Gehäuse 15 der Mikropumpe 10, durch die das zu bewegende Fluid, insbesondere das Blut, ausströmt.

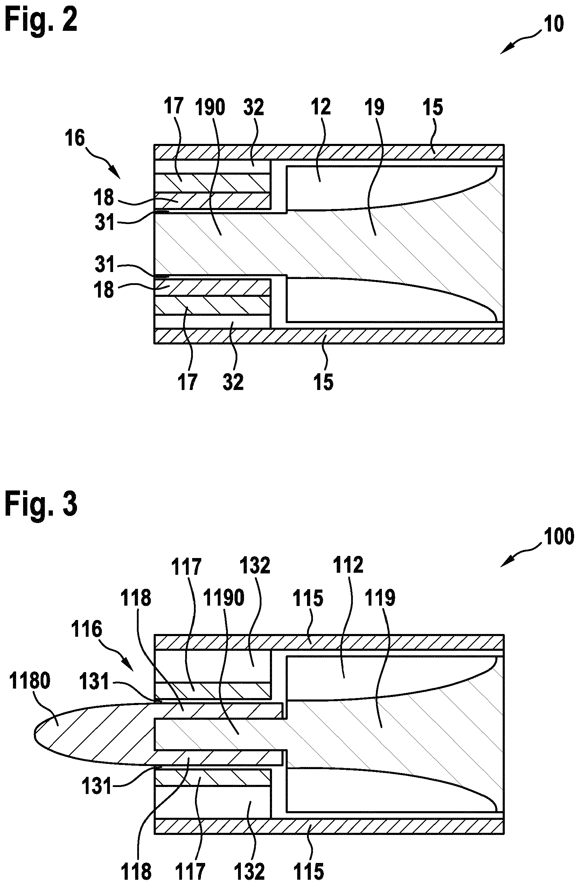

Fig. 2 zeigt den Bereich des Gleitlagers 16 für die radiale Lagerung des Laufradzapfens 190 des Laufrades 19 mit den Laufradblättern 12 als Bestandteil einer Mikropumpe 10 gemäß Fig. 1 in einem schematisierten Längsschnitt. Der Laufradzapfen 190 ist rotierend innerhalb der Lagerbuchse 18 gelagert, wobei zwischen Laufradzapfen 190 und Lagerbuchse 18 ein schmaler Lagerspalt 31 vorgesehen ist. Die Lagerbuchse 18 befindet sich innerhalb des Lagersterns 17. Die Bereiche 32 deuten die Öffnungen des Lagersterns 17 an, durch die das Fluid, insbesondere das Blut, fließen kann.

Fig. 3 zeigt demgegenüber eine bevorzugte Ausführungsform einer gekapselten Mikropumpe 100 gemäß der Erfindung, wobei hier ebenfalls der Bereich des Gleitlagers 116 gezeigt ist. Der hier gezeigte Ausschnitt der erfindungsgemäßen gekapselten Mikropumpe 100 zeigt den Laufradzapfen 1190 des Laufrades 119 mit den Laufradblättern 112, wobei der Laufradzapfen 1190 in dem Gleitlager 116 rotierend gelagert ist. Innerhalb des Gehäuses 150 der Mikropumpe 100 befindet sich im Bereich des radialen Gleitlagers 116 der Lagerstern 117. Im Bereich des Gleitlagers 116 ist der Laufradzapfen 1190 verjüngt. Die Verjüngung 1190 ist von einer Ummantelung 118 umgeben. Diese Ummantelung 118 besteht aus einem anderen Material als der Lagerstern 117. Insbesondere können die Ummantelung 118 aus PEEK und der Lagerstern 117 aus einem metallischen Werkstoff, insbesondere Titan, gefertigt sein.

Zwischen der Ummantelung 118 und dem Lagerstern 117 befindet sich ein schmaler Lagerspalt 131. Somit rotiert der mit PEEK ummantelte Laufradzapfen 1190 in der zentralen Aussparung des Lagersterns 117, wobei die tribologisch vorteilhafte Materialpaarung von beispielsweise PEEK und Titan realisiert ist. Im Vergleich mit einem Gleitlager nach Fig. 2 wird bei der erfindungsgemäßen Lösung die Lagerbuchse 18 durch die Ummantelung 118 gewissermaßen ersetzt, wobei der Gesamtdurchmesser nicht verändert wird. Durch diese Maßnahme kann der erforderliche Platzbedarf der Lagerbuchse 18 für andere Zwecke genutzt werden, und zwar können hierdurch die Öffnungen 132 innerhalb des Lagersterns 117, die für den Fluiddurchfluss vorgesehen sind, vergrößert werden. Bei gleichen funktionalen Lagerabmessungen (z. B. Lagerdurchmesser 1 mm, Lagerspalt 10 µm und Wandstärke der Ummantelung 0,25 mm) steht somit mehr Querschnitt für die Strömung zur Verfügung.

In besonderer bevorzugter Weise kann darüber hinaus die Ummantelung 118 in Form einer Kappe 1180 realisiert sein, die die Ummantelung 118 stromaufwärts verlängert und durch entsprechende Formung strömungstechnische Vorteile bietet. Insbesondere kann die Form der Kappe 1180 einen stromaufwärts kleiner werdenden Durchmesser aufweisen, insbesondere in einer konischen oder halbellipsoiden Form. Hierdurch kann eine bessere Strömungsführung um das Lager 116 herum realisiert werden, wodurch zusätzlich Druckverluste reduziert und die Effizienz der Mikropumpe 100 gesteigert werden.

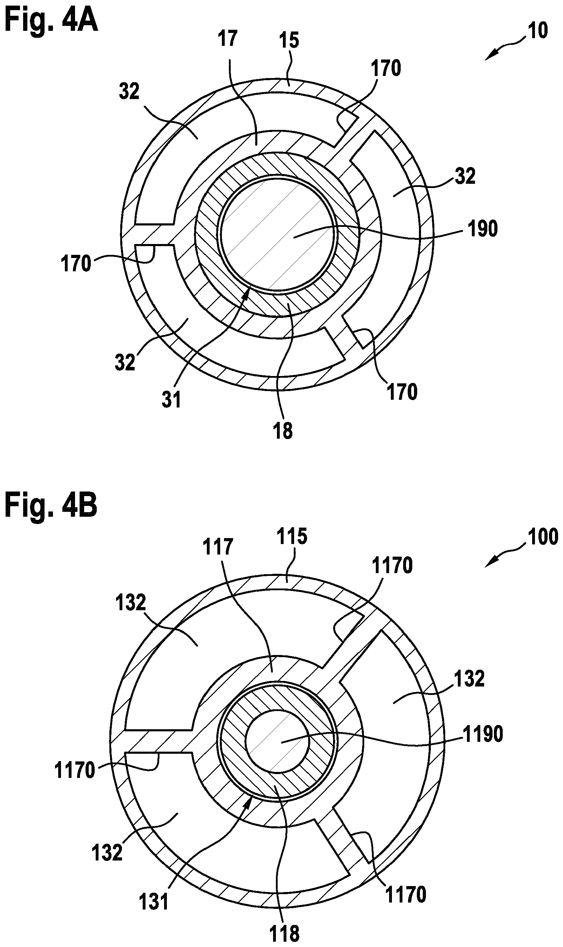

Fig. 4 illustriert die Ausgestaltung der erfindungsgemäßen Mikropumpe 100 im Bereich des Gleitlagers (Teilfigur 4B) im Vergleich mit dem Gleitlager einer Mikropumpe 10 nach Fig. 1 (Teilfigur 4A) im Querschnitt. Die Darstellung in Teilfigur A zeigt das Gleitlager mit dem Laufradzapfen 19, der innerhalb der Lagerbuchse 18, getrennt durch den Lagerspalt 31, rotierend gelagert ist. Die Lagerbuchse 18 befindet sich im Inneren des Lagersterns 17, der über die Lagersternstreben 170 innerhalb des Gehäuses 15 der Mikropumpe 10 befestigt ist. Zwischen den einzelnen Lagersternstreben 170 befindet sich der Raum 32, der von dem Fluid durchflossen werden kann. Im Vergleich mit der erfindungsgemäßen Ausgestaltung in Teilfigur B wird deutlich, dass der entsprechende Bereich 132 bei der erfindungsgemäßen Lösung wesentlich vergrößert ist. Die Teilfigur B zeigt hierbei den verjüngten Bereich des Laufradzapfens 1190, der direkt von der Ummantelung 118 aus einem anderen Material umgeben ist. Zwischen der Ummantelung 118 und dem Inneren des Lagersterns 117 (zentrale Aussparung des Lagersterns 17) befindet sich der schmale Lagerspalt 131. Das Innere des Lagersterns 117 ist über die Lagersternstreben 1170 mit dem Gehäuse 115 der Mikropumpe 100 verbunden. Durch diese Ausgestaltung ist es möglich, den Bereich 132 für den Fluiddurchfluss im Vergleich mit Gleitlagern gemäß Fig. 1 wesentlich zu vergrößern. Die erfindungsgemäße Mikropumpe 100 erzeugt daher deutlich weniger Druckverluste im Bereich des stromaufwärts gelegenen radialen Gleitlagers des Laufradzapfens.

Eine solche Mikropumpe kann mit besonderem Vorteil beispielsweise als Blutpumpe für ein Herzunterstützungssystem eingesetzt werden.

## Patentansprüche

1. Gekapselte Mikropumpe (100) mit integriertem Motor und wenigstens einem Laufrad (119) zur Erzeugung eines Fluidflusses innerhalb eines Gehäuses (115) der Mikropumpe, wobei das Laufrad (119) ein radiales Gleitlager (116) mit einem Lagerstern (117, 1170) zur Lagerung eines Laufradzapfens (1190) des Laufrades (119) innerhalb des Gehäuses (115) aufweist, wobei der Laufradzapfen (1190) eine Ummantelung (118) aus einem anderen Material als der Lagerstern (117, 1170) aufweist, **dadurch gekennzeichnet, dass** die Ummantelung (118) des Laufradzapfens (1190) eine Kappe bildet, die über den Lagerstern (117) hinaus eine Verlängerung (1180) aufweist.

2. Gekapselte Mikropumpe nach Anspruch 1, **dadurch gekennzeichnet, dass** der Laufradzapfen (1190) und der Lagerstern (117, 1170) jeweils aus einem metallischen Werkstoff besteht und dass die Ummantelung (118) des Laufradzapfens (1190) aus einem Kunststoff besteht.

3. Gekapselte Mikropumpe nach Anspruch 1 oder Anspruch 2, **dadurch gekennzeichnet, dass** der Laufradzapfen (1190) und der Lagerstern (117, 1170) aus Titan und die Ummantelung (118) des Laufradzapfens (1190) aus Polyetheretherketon besteht.

4. Gekapselte Mikropumpe nach einem der vorhergehenden Ansprüche, **dadurch gekennzeichnet, dass** der Laufradzapfen (1190) im Bereich der Ummantelung (118) eine Verjüngung aufweist.

5. Gekapselte Mikropumpe nach einem der vorhergehenden Ansprüche, **dadurch gekennzeichnet, dass** die Verlängerung (1180) sich verjüngt.

6. Gekapselte Mikropumpe nach einem der vorhergehenden Ansprüche, **dadurch gekennzeichnet, dass** die Verlängerung (1180) konisch oder halbellipsoid ist.

7. Gekapselte Mikropumpe nach einem der vorhergehenden Ansprüche, **dadurch gekennzeichnet, dass** die Mikropumpe (100) eine Blutpumpe ist.

8. Gekapselte Mikropumpe nach einem der vorhergehenden Ansprüche, **dadurch gekennzeichnet, dass** die gekapselte Mikropumpe (100) Bestandteil eines ventrikulären Herzunterstützungssystems ist.

9. Gekapselte Mikropumpe nach einem der vorhergehenden Ansprüche, **dadurch gekennzeichnet, dass** der Außendurchmesser der gekapselten Mikropumpe (100) 10 mm oder weniger beträgt.

## Claims

1. Encapsulated micropump (100) having an integrated motor and at least one impeller (119) for generating a fluid flow within a housing (115) of the micropump, the impeller (119) having a radial plain bearing (116) with a bearing star (117, 1170) for mounting an impeller journal (1190) of the impeller (119) within the housing (115), wherein the impeller journal (1190) has a casing (118) made of a different material than the bearing star (117, 1170), **characterized in that** the casing (118) of the impeller journal (1190) forms a cap which has an extension (1180) beyond the bearing star (117).

2. Encapsulated micropump according to claim 1, **characterized in that** the impeller journal (1190) and the bearing star (117, 1170) each consist of a metallic material and **in that** the casing (118) of the impeller journal (1190) consists of a plastic material.

3. Encapsulated micropump according to claim 1 or claim 2, **characterized in that** the impeller journal (1190) and the bearing star (117, 1170) are made of titanium and the casing (118) of the impeller journal (1190) is made of polyetheretherketone.

4. Encapsulated micropump according to one of the preceding claims, **characterized in that** the impeller journal (1190) has a taper in the region of the casing (118).

5. Encapsulated micropump according to one of the preceding claims, **characterized in that** the extension (1180) is tapered.

6. Encapsulated micropump according to one of the preceding claims, **characterized in that** the extension (1180) is conical or semi-ellipsoidal.

7. Encapsulated micropump according to one of the preceding claims, **characterized in that** the micropump (100) is a blood pump.

8. Encapsulated micropump according to one of the preceding claims, **characterized in that** the encapsulated micropump (100) is a component of a ventricular cardiac support system.

9. Encapsulated micropump according to one of the preceding claims, **characterized in that** the outer diameter of the encapsulated micropump (100) is 10 mm or less.

## Revendications

1. Micropompe (100) encapsulée comportant un moteur intégré et au moins une roue mobile (119) permettant de générer un flux de fluide à l'intérieur d'un boîtier (115) de la micropompe, dans laquelle la roue mobile (119) présente un palier lisse radial (116) comportant une étoile de palier (117, 1170) permettant de supporter un tourillon de roue mobile (1190) de la roue mobile (119) à l'intérieur du boîtier (115), dans laquelle le tourillon de roue mobile (1190) présente une enveloppe (118) en un autre matériau que celui de l'étoile de palier (117, 1170), **caractérisée en ce que** l'enveloppe (118) du tourillon de roue mobile (1190) forme un capuchon qui présente un prolongement (1180) au-delà de l'étoile de palier (117).

2. Micropompe encapsulée selon la revendication 1, **caractérisée en ce que** le tourillon de roue mobile (1190) et l'étoile de palier (117, 1170) sont respectivement constitués d'une matière première métallique **et en ce que** l'enveloppe (118) du tourillon de roue mobile (1190) est constituée d'une matière plastique.

3. Micropompe encapsulée selon la revendication 1 ou la revendication 2, **caractérisée en ce que** le tourillon de roue mobile (1190) et l'étoile de palier (117, 1170) sont constitués de titane et l'enveloppe (118) du tourillon de roue mobile (1190) est constituée de polyétheréthercétone.

4. Micropompe encapsulée selon l'une des revendications précédentes, **caractérisée en ce que** le tourillon de roue mobile (1190) présente un rétrécissement dans la zone de l'enveloppe (118).

5. Micropompe encapsulée selon l'une des revendications précédentes, **caractérisée en ce que** le prolongement (1180) se rétrécit.

6. Micropompe encapsulée selon l'une des revendications précédentes, **caractérisée en ce que** le prolongement (1180) est conique ou semi-ellipsoïdal.

7. Micropompe encapsulée selon l'une des revendications précédentes, **caractérisée en ce que** la micropompe (100) est une pompe à sang.

8. Micropompe encapsulée selon l'une des revendications précédentes, **caractérisée en ce que** la micropompe (100) encapsulée fait partie d'un système d'assistance cardiaque ventriculaire.

9. Micropompe encapsulée selon l'une des revendications précédentes, **caractérisée en ce que** le diamètre extérieur de la micropompe (100) encapsulée est de 10 mm ou moins.
